(19) **Europäisches Patentamt**
**European Patent Office**
**Office européen des brevets**

(11) **EP 1 941 911 A1**

(12) **EUROPEAN PATENT APPLICATION**
published in accordance with Art. 153(4) EPC

(43) Date of publication:
09.07.2008 Bulletin 2008/28

(21) Application number: 06822434.4

(22) Date of filing: 27.10.2006

(51) Int Cl.:
*A61K 45/06* (2006.01)    *A61K 31/439* (2006.01)
*A61K 31/50* (2006.01)    *A61P 19/02* (2006.01)
*A61P 29/00* (2006.01)

(86) International application number:
**PCT/JP2006/321466**

(87) International publication number:
**WO 2007/049732 (03.05.2007 Gazette 2007/18)**

(84) Designated Contracting States:
**AT BE BG CH CY CZ DE DK EE ES FI FR GB GR
HU IE IS IT LI LT LU LV MC NL PL PT RO SE SI
SK TR**
Designated Extension States:
**AL BA HR MK RS**

(30) Priority: 28.10.2005 US 730841 P

(71) Applicant: Kowa Company, Ltd.
Naka-ku
Nagoya-shi, Aichi-ken 460-8625 (JP)

(72) Inventors:
• TABUNOKI, Yuichiro
Tokyo 2030051 (JP)
• KOSHI, Tomoyuki
Shiki-shi, Saitama 3530006 (JP)

(74) Representative: Blodig, Wolfgang
Wächtershäuser & Hartz
Patentanwälte
Weinstrasse 8
80333 München (DE)

(54) **METHOD FOR PREVENTION AND/OR TREATMENT OF RHEUMATOID ARTHRITIS**

(57) The invention provides a method for the prevention and/or treatment of rheumatoid arthritis, **characterized in that** the method comprises administering an IL-1β inhibitor and a calcineurin inhibitor, and a preventive and/or therapeutic medicine for rheumatoid arthritis including an IL-1β inhibitor and a calcineurin inhibitor in combination.

According to the invention, there can be provided a medicine and method for treating rheumatoid arthritis which exhibits suppressed side effects and excellent potency for suppression of arthritis.

**Description**

Technical Field

The present invention relates to a method for the prevention and/or treatment of rheumatoid arthritis.

Background Art

Rheumatoid arthritis is a disease which involves inflammation in many joints, concomitant with swelling and pain. When rheumatoid arthritis has progressed for a long period of time, the patient suffers irreversible deformity in the joints and functional disorders, and quality of life (QOL) of the patient is deteriorated considerably. In Japan, 0.6% of the total population and 1% of the population over age 30 suffer rheumatoid arthritis. With the progressive aging of society in recent years, elderly patients of rheumatoid arthritis have gradually increased in number.

Rheumatoid arthritis progresses through the following four stages. In the initial stage, joint pain and arthritis are observed, but the patient cannot be definitely diagnosed as suffering rheumatoid arthritis. In the early stage, the patient can be definitely diagnosed as suffering rheumatoid arthritis, but exhibits no or slight irreversible deformity (early stage of rheumatoid arthritis generally refers to a stage for 1 to 2 years from the onset thereof). In the progressive stage, the patient exhibits irreversible deformity and significant systemic symptoms, including fatigue, low-grade fever, and weight loss. In the late stage, arthritis has been almost sedated, but irreversible deformity such as deformity/contracture remains, resulting in pain and functional disorders as predominant symptoms. The method for the treatment varies depending on the corresponding stage of rheumatoid arthritis. The onset mechanism of rheumatoid arthritis has not yet been elucidated, although some studies report a relationship between the onset and a factor such as a hereditary factor or an acquired factor (an infectious disease). Therefore, complete prevention and curing of rheumatoid arthritis cannot be attained.

Thus, at present, treatment goals of rheumatoid arthritis are in early establishment of diagnosis as rheumatoid arthritis and suppressing inflammation caused by rheumatoid arthritis as soon as and as effectively as possible, whereby expression or progress of irreversible deformity is prevented so as to enhance QOL of patients from physical, mental, and social aspects. In this connection, upon the treatment of rheumatoid arthritis, the patients are well instructed in advance in terms of characteristics and the treatment of the disease, and receive a variety of treatment means such as physical therapy, kinesitherapy, drug therapy, and surgery.

In drug therapy, drugs such as non-steroidal antiinflammatory drugs (NSAIDs), disease-modifying anti-rheumatic drugs (DMARDs), and steroids are employed in clinical settings. Recently, a biologics such as an antibody against an inflammatory cytokine as a target is also employed (Non-Patent Document 1).

A calcineurin inhibitor has been conventionally employed as an immunosuppressor. Known calcineurin inhibitors are cyclosporin (see Patent Document 1), tacrolimus (see Patent Document 2), ISA-247 (see Patent Document 3), 7-oxabicyclo[2.2.1]heptane-2,3-dicarboxylate derivatives (see Patent Document 4), INCA compounds (see Non-Patent Document 2), etc. Among calcineurin inhibitors, tacrolimus has been approved as a new DMARD in recent years (April, 2005). Tacrolimus has an action mechanism which differs from that of a conventional drug for the treatment of rheumatoid arthritis. Therefore, tacrolimus is a candidate as an effective therapeutic drug for rheumatoid arthritis of patients who have not sufficiently cured by a conventional drug. However, tacrolimus must be used carefully, due to adverse side effects such as renal disorders, hypertension, and diabetes.

In many diseases such as rheumatoid arthritis, osteoarthritis, osteoporosis, inflammatory colitis, immune deficiency syndrome, ichorrhemia, hepatitis, nephritis, ischemic diseases, insulin-dependent diabetes, arterial sclerosis, Perkinson's disease, Alzheimer's disease, and leukemia, stimulation of production of interleukin 1β (IL-1β), which is an inflammatory cytokine, is observed. IL-1β is known to induce synthesis of enzymes which are conceived to involve inflammation; e.g., collagenase, COX, and PLA2, and to cause articular damage very similar to that caused by rheumatoid arthritis when intra-articularly injected to animals. Thus, IL-1β inhibitors are studied and developed to serve as drugs for the treatment of inflammatory diseases. Specifically, hitherto, there have been known bio-substances such as IL-1 receptor antagonist (see Non-Patent Document 3) and IL-1β antibodies (see Patent Documents 5, 6, and 7); and low-molecular-weight compounds such as T-614 (see Non-Patent Document 4); S-2474 (see Non-Patent Document 5), 2-benzyl-5-(4-chlorophenyl)-6-[4-(methylthio)phenyl]-2H-pyridazin-3-one (see Patent Document 8); FR133605 (see Non-Patent Document 6), a halomethylamide derivative (see Patent Document 9), a pyrrolidine derivative (see Patent Document 10), and aminobenzophenone derivatives (see patent Documents 11, 12, and 13).

Pathological conditions of rheumatoid arthritis vary considerably among patients in terms of age, stage, complications, side effects, QOL, etc. Hitherto, an ultimate therapeutic drug therefor has not yet been developed. Even though a therapeutic drug can control the disease condition, in some cases, the effect of the drug is suddenly lost, in a phenomenon called "escape phenomenon." Under such circumstances, when drug therapy is performed, change of drugs and combined use of drugs are generally employed. Thus, clinical studies on the mode of use of such drugs are carried out extensively.

Meanwhile, hitherto, nothing has been known the effect of combined use of a calcineurin inhibitor and an IL-1β inhibitor.

Patent Document 1: WO 92/011860, pamphlet
Patent Document 2: WO 00/007594, pamphlet
Patent Document 3: WO 99/018120, pamphlet
Patent Document 4: JP-A-2000-309590
Patent Document 5: WO 01/053353, pamphlet
Patent Document 6: WO 02/016436, pamphlet
Patent Document 7: WO 04/072116, pamphlet
Patent Document 8: WO 99/025697, pamphlet
Patent Document 9: WO 95/029672, pamphlet
Patent Document 10: WO 90/225458, pamphlet
Patent Document 11: WO 01/005745, pamphlet
Patent Document 12: WO 01/042189, pamphlet
Patent Document 13: WO 01/005751, pamphlet
Non-Patent Document 1: Arthritis & Rheumatism 46, pp 328-346, 2002
Non-Patent Document 2: Proc Natl Acad Sci USA. 101, pp. 7554-7559, 2004
Non-Patent Document 3: Arthritis & Rheumatism 42, pp. 498-506, 1999
Non-Patent Document 4: J. Pharmacobio-Dyn. 11, pp. 649-655, 1992
Non-Patent Document 5: YAKUGAKU ZASSHI 123, pp. 323-330, 2003
Non-Patent Document 6: J. Rheumatol. 23, pp. 1778-1783, 1996

Disclosure of the Invention

Thus, an object of the present invention is to provide a medicine and method for the prevention and/or treatment of rheumatoid arthritis, which medicine and method exhibits suppressed side effects and excellent potency for suppression of arthritis.
Yet another object of the invention is to provide, for avoiding the escape phenomenon, an alternative drug therapy and combinatory use means.
In view of the foregoing, the present inventors have conducted extensive studies, and have found that use in combination of an IL-1β inhibitor and a calcineurin inhibitor provides an excellent effect of preventing arthritis. The present invention has been accomplished on the basis of this finding.
Accordingly, the present invention provides a preventive and/or therapeutic medicine for rheumatoid arthritis, the medicine comprising an IL-1β inhibitor and a calcineurin inhibitor in combination.
The present invention also provides a method for the prevention and/or treatment of rheumatoid arthritis, characterized in that the method comprises administering an IL-1β inhibitor and a calcineurin inhibitor.
The present invention also provides use of an IL-1β inhibitor and a calcineurin inhibitor for production of a preventive and/or therapeutic medicine for rheumatoid arthritis.
Since the medicine according to the present invention exhibits suppressed side effects and excellent potency for suppression of arthritis, the medicine is useful for the prevention and/or treatment of rheumatoid arthritis.

Brief Description of the Drawing

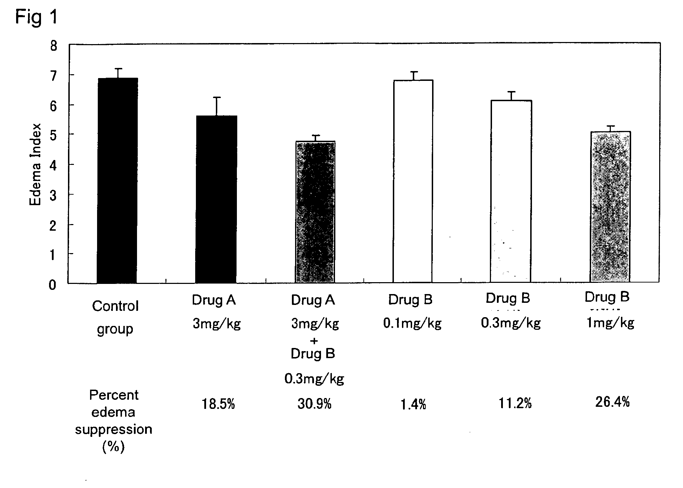

[Fig. 1] A graph showing Edema indices of rats of a collagen-induced arthritis model measured, the rats being divided into a control group (group of no drug administration), an administration group (2-benzyl-5-(4-chlorophenyl)-6-[4-(methylthio)phenyl]-2H-pyridazin-3-one (Drug A): 3 mg/kg), a combined administration group (Drug A: 3 mg/kg and tacrolimus (calcineurin inhibitor, Drug B): 0.3 mg/kg), and administration groups (Drug B: 0.1, 0.3, and 1 mg/kg).

Best Modes for Carrying Out the Invention

Examples of the IL-1β inhibitor employed in the present invention include biogenic substances such as IL-1 receptor antagonist and IL-1β antibodies; and low-molecular-weight compounds such as T-614, S-2474, 2-benzyl-5-(4-chlorophenyl)-6-[4-(methylthio)phenyl]-2H-pyridazin-3-one, FR133605, a halomethylamide derivative, a pyrrolidine derivative, and an aminobenzophenone derivative. Of these, 2-benzyl-5-(4-chlorophenyl)-6-[4-(methylthio)phenyl]-2H-pyridazin-3-one is particularly preferred.

2-benzyl-5-(4-chlorophenyl)-6-[4-(methylthio)phenyl]-2H-pyridazin-3-one employed in the present invention may be produced through the method disclosed in WO 99/025697 (pamphlet) or a similar method. Specifically, p-chlorophenylacetic acid is reacted with thioanisole in the presence of a condensing agent such as polyphosphoric acid, to thereby form 2-(4-chlorophenyl)-4'-(methylthio)acetophenone. The thus-formed 2-(4-chlorophenyl)-4'-(methylthio)acetophenone is reacted with a base such as potassium t-butoxide in tetrahydrofuran, followed by adding ethyl bromoacetate to the reaction system, to thereby form ethyl 2-(4-chlorophenyl)-4-[4-(methylthio)phenyl]-4-oxobutanate. The thus-formed ethyl 2-(4-chlorophenyl)-4-[4-(methylthio)phenyl]-4-oxobutanate is reacted with hydrazine hydrate in ethanol, to thereby form 5-(4-chlorophenyl)-6-[4-(methylthio)phenyl]-4,5-dihydro-2H-pyridazin-3-one. The thus-formed 5-(4-chlorophenyl)-6-[4-(methylthio)phenyl]-4,5-dihydro-2H-pyridazin-3-one is reacted with benzyl bromide in a solvent such as N,N-dimethylformamide in the presence of a base such as potassium carbonate, to thereby yield 2-benzyl-5-(4-chlorophenyl)-6-[4-(methylthio)phenyl]-2H-pyridazin-3-one.

Examples of the calcineurin inhibitor employed in the present invention include cyclosporin, tacrolimus, ISA-247, 7-oxabicyclo[2.2.1]heptane-2,3-dicarboxylate derivatives, and INCA compounds. Among them, tacrolimus is particularly preferred. A commercial tacrolimus product such as a product of Astellas Pharma Inc. may also be employed in the invention.

As shown in the Example given hereinbelow, both-hindlimb edema can be synergistically suppressed through administration, in combination, of an IL-1β inhibitor (e.g., 2-benzyl-5-(4-chlorophenyl)-6-[4-(methylthio)phenyl]-2H-pyridazin-3-one) and a calcineurin inhibitor (e.g., tacrolimus), whereby arthritis can be suppressed. Therefore, a medicine comprising an IL-1β inhibitor and a calcineurin inhibitor in combination is useful for a preventive and/or therapeutic medicine for rheumatoid arthritis, particularly for rheumatoid arthritis involving inflammation of a joint.

In the method according to the present invention for the prevention and/or treatment of rheumatoid arthritis and the preventive and/or therapeutic medicine according to the present invention for rheumatoid arthritis, the mass ratio of IL-1β inhibitor to calcineurin inhibitor is preferably 300 : 1 to 1 : 3, particularly preferably 100 : 1 to 3 : 1, from the viewpoint of a synergistic arthritis suppression action.

The preventive and/or therapeutic medicine according to the present invention for rheumatoid arthritis may be provided as a kit including a medicine containing an IL-1β inhibitor and a medicine containing a calcineurin inhibitor. Alternatively, the medicine according to the present invention may be provided as a combination preparation containing an IL-1β inhibitor and a calcineurin inhibitor.

In other words, the IL-1β inhibitor and calcineurin inhibitor of the present invention may be administered simultaneously or separately with a predetermined interval, or administered as a combination preparation.

No particular limitation is imposed on the mode of administering the medicine of the present invention, and the mode may be appropriately selected in accordance with the purpose of the treatment. In oral administration, tablets, capsules, granules, film-coated drugs, powders, and syrups may be employed. In parenteral administration, injections, suppositories, inhalations, percutaneous drugs, eye drops, and nasal drops may be employed. Of these, oral administration is preferred.

The pharmaceutical products suited for the above modes of administration may appropriately be employed with the pharmacologically acceptable carriers as exemplified below:

vehicles and bulking agents such as starch, lactose, sucrose, mannitol, and silicic acid; disintegrants such as agar, calcium carbonate, potato or tapioca starch, alginic acid, and specific complex silicate salts; binders such as hydroxypropyl methyl cellulose, alginate salts, gelatin, polyvinylpyrrolidone, sucrose, and acacia; lubricants such as talc, calcium stearate, magnesium stearate, solid polyethylene glycols, sodium lauryl sulfate, and mixtures thereof; diluents such as lactose and corn starch; buffers such as organic acids (e.g., citric acid, phosphoric acid, tartaric acid, and lactic acid), inorganic acids (e.g., hydrochloric acid), alkali hydroxides (e.g., sodium hydroxide and potassium hydroxide), and amines (e.g., triethanolamine, diethanolamine, and diisopropanolamine); antiseptic agents such as p-oxybenzoate esters and benzalkonium chloride; emulsifying agents such as anionic surfactants (e.g., calcium stearate, magnesium stearate, and sodium lauryl sulfate), cationic surfactants (e.g., benzalkonium chloride, benzetonium chloride, and cetylpyridinium chloride), and nonionic surfactants (e.g., glyceryl monostearate, sucrose fatty acid esters, polyoxyethylene-hardened castor oil, polyoxyethylene sorbitan fatty acid esters, polyoxyethylene fatty acid esters, and polyoxyethylene alkyl ethers); and stabilizing agents such as sodium sulfite, sodium bisulfite, dibutylhydroxytoluene, butylhydroxyanisole, and EDTA. In addition, additives such as an odor-suppressor, a dispersant, a preservative, and a flavoring may appropriately be used in accordance with needs.

Among the ingredients of the medicine according to the present invention, the IL-1β inhibitor (e.g., 2-benzyl-5-(4-chlorophenyl)-6-[4-(methylthio)phenyl]-2H-pyridazin-3-one) is administered at a dose which is appropriately selected in accordance with the body weight, age, sex, condition, etc. of a patient in need thereof. Generally, the daily dose per adult is 2 to 320 mg, preferably 4 to 160 mg. The calcineurin inhibitor (e.g., tacrolimus) is administered at a dose which is appropriately selected in accordance with the body weight, age, sex, condition, etc. of a patient in need thereof. Generally, the daily dose per adult is 0.06 to 5 mg, preferably 1.5 to 3 mg. The ingredients may be administered in a single dose per day, or in two or more daily doses in a divided manner.

Examples

The present invention will next be described in more detail by the following examples, which should not be construed as limiting the invention thereto.

Example 1

The effect on suppressing both-hindlimb edema of 2-benzyl-5-(4-chlorophenyl)-6-[4-(methylthio)phenyl]-2H-pyridazin-3-one (synthesized through the aforementioned method) and that of tacrolimus (Prograf (injection: 5 mg), product of Fujisawa Pharmaceutical Co., Ltd.) were determined both in the cases of combined administration and solo administration through the following procedure (by use of rats of a collagen-induced arthritis model) (FDA, CBER, CDER, CDRH: Guidance for industry - Clinical development programs for drugs, devices, and biological products for the treatment of rheumatoid arthritis (RA)-.(1999)). Lewis female rats (LEW/Crj) (obtained from Charles River Laboratories Japan, Inc.) were employed as test animals.

For each 8-week-old LEW/Crj rat, the volume of a portion from the ankle to the toe tip of each hindlimb was measured by means of a plethysmometer for small animals (TK-101CMP, product of Unicom), and a total of the two volumes was employed as a volume of the hindlimbs (hereinafter referred to as both-hindlimb volume) at the start of the test (hereinafter referred to as Pre value). By use of the Pre value as an index, the rats were divided into groups which are alike from group to group, through one-parameter-based block randomization.

The collagen emulsion for sensitization employed for inducing arthritis in rats was prepared by homogenizing a 0.3% Type II collagen liquid (product of Collagen Research Center), adjuvant peptide (product of Peptide Institute, Inc.), and adjuvant incomplete Freund (product of DIFCO) by means of a Handy Micro Homogenizer (product of Microtec Nition) under cooling with ice. The thus-prepared emulsion was intracutaneously injected to 10 sites in the dorsum of each rat at 0.1 mL/site, to thereby initially sensitize the rat. Seven days after initial sensitization, the same emulsion (0.12 mL) was intracutaneously injected to the tail head of the rat for booster sensitization.

Administration of one drug or two drugs was carried out the day after initial sensitization to day 26. To the 2-benzyl-5-(4-chlorophenyl)-6-[4-(methylthio)phenyl]-2H-pyridazin-3-one solo administration group, the drug was orally administered twice; in the morning (9:00 to 11:00) and in the evening (15:30 to 17:30) at a dose of 3 mg/kg. To the tacrolimus sole administration group, the drug was orally administered once in the afternoon (11:30 to 13:30) at a dose of 0.1, 0.3, or 1 mg/kg. To the combined administration group (2-benzyl-5-(4-chlorophenyl)-6-[4-(methylthio)phenyl]-2H-pyridazin-3-one and tacrolimus), 2-benzyl-5-(4-chlorophenyl)-6-[4-(methylthio)phenyl]-2H-pyridazin-3-one was orally administered in the morning (9:00 to 11:00) and in the evening (15:30 to 17:30) at a dose of 3 mg/kg, and tacrolimus was orally administered in the afternoon (11:30 to 13:30) at a dose of 0.3 mg/kg.

14 days, 18 days, 22 days, and 26 days after initial sensitization, both-hindlimb volume was measured. Volume of both-hindlimb edema was calculated by subtracting the Pre value from the thus-measured value. The sum of the volumes of both-hindlimb edema at days 14, 18, 22, and 26 after initial sensitization was employed as an Edema Index, which was used as the index for assessing the potency of the drug(s).

Table 1 and Fig. 1 show Edema Index of the groups of rats, the groups being the 2-benzyl-5-(4-chlorophenyl)-6-[4-(methylthio)phenyl]-2H-pyridazin-3-one solo administration group, the tacrolimus solo administration group, and the combined administration group. The Edema Index of each group is an average of the indices obtained from 6 to 12 rats $\pm$ a standard error. Percent edema suppression represents a value obtained from the relationship:

```
100×[(average both-hindlimb edema volume of the control
group) - (average both-hindlimb edema volume of each
administration group)]/(average both-hindlimb edema volume of
the control group). Relative factor represents a ratio of
(average both-hindlimb edema volume of each administration
group)/(average both-hindlimb edema volume of the control
group).
```

As a result, solo administration of 2-benzyl-5-(4-chlorophenyl)-6-[4-(methylthio)phenyl]-2H-pyridazin-3-one and that of tacrolimus (up to 1 mg/kg) do not result in potent Edema Index suppression effects.

In contrast, combined administration of both drugs exhibits a potent effect on reducing Edema Index, and its effect is higher than that obtained through administration of tacrolimus 1 mg/kg. The relative factor of Edema Index of the group of combined administration of both drugs is smaller than the product of the relative factors of the solo administration groups, indicating that a clear synergistic effect can be attained through combined administration.

[Table 1]

| Edema Index | | | |
|---|---|---|---|
| Tested drugs | Edema Index (mL) | Percent edema suppression (%) | Relative factor |
| Control group | $6.85\pm0.33$ | | |
| 2-benzyl-5-(4-chlorophenyl)-6-[4-(methylthio)phenyl]-2H-pyridazin-3-one solo administration group (3 mg/kg) | $5.58\pm0.63$ | 19 | 0.81 |
| 2-benzyl-5-(4-chlorophenyl)-6-[4-(methylthio)phenyl]-2H-pyridazin-3-one (3 mg/kg) and tacrolimus (0.3 mg/kg) combined administration group | $4.73\pm0.20$ | 31 | 0.69 |
| Tacrolimus solo administration group (0.1 mg/kg) | $6.75\pm0.31$ | 1 | 0.99 |
| Tacrolimus solo administration group (0.3 mg/kg) | $6.09\pm0.30$ | 11 | 0.89 |
| Tacrolimus solo administration group (1 mg/kg) | $5.04\pm0.20$ | 26 | 0.74 |
| Product of relative factors of solo administration groups: $0.81\times0.89=0.72$<br>Both-hindlimb edema volume of each group is an average of the volumes obtained from 6 to 12 rats $\pm$ a standard error. | | | |

**Claims**

1.   A preventive and/or therapeutic medicine for rheumatoid arthritis, the medicine comprising an IL-1β inhibitor and a calcineurin inhibitor in combination.

2.   A preventive and/or therapeutic medicine for rheumatoid arthritis as described in claim 1, wherein the rheumatoid arthritis involves inflammation of a joint.

3. A preventive and/or therapeutic medicine for rheumatoid arthritis as described in claim 1 or 2, wherein the IL-1β inhibitor is 2-benzyl-5-(4-chlorophenyl)-6-[4-(methylthio)phenyl]-2H-pyridazin-3-one.

4. A preventive and/or therapeutic medicine for rheumatoid arthritis as described in any one of claims 1 to 3, wherein the calcineurin inhibitor is tacrolimus.

5. A preventive and/or therapeutic medicine for rheumatoid arthritis as described in any one of claims 1 to 4, wherein the medicine is for peroral administration.

6. A method for the prevention and/or treatment of rheumatoid arthritis, **characterized in that** the method comprises administering an IL-1β inhibitor and a calcineurin inhibitor.

7. The method for the prevention and/or treatment of rheumatoid arthritis as described in claim 6, wherein the rheumatoid arthritis involves inflammation of a joint.

8. The method for the prevention and/or treatment of rheumatoid arthritis as described in claim 6 or 7, wherein the IL-1β inhibitor is 2-benzyl-5-(4-chlorophenyl)-6-[4-(methylthio)phenyl]-2H-pyridazin-3-one.

9. The method for the prevention and/or treatment of rheumatoid arthritis as described in any one of claims 6 to 8, wherein the calcineurin inhibitor is tacrolimus.

10. The method for the prevention and/or treatment of rheumatoid arthritis as described in any one of claims 6 to 9, wherein said administering is carried out through oral administration.

11. Use of an IL-1β inhibitor and a calcineurin inhibitor for production of a preventive and/or therapeutic medicine for rheumatoid arthritis.

12. Use as described in claim 11 for production of a preventive and/or therapeutic medicine for rheumatoid arthritis, wherein the rheumatoid arthritis involves inflammation of a joint.

13. Use as described in claim 11 or 12 for production of a preventive and/or therapeutic medicine for rheumatoid arthritis, wherein the IL-1β inhibitor is 2-benzyl-5-(4-chlorophenyl)-6-[4-(methylthio)phenyl]-2H-pyridazin-3-one.

14. Use as described in any one of claims 11 to 13 for production of a preventive and/or therapeutic medicine for rheumatoid arthritis, wherein the calcineurin inhibitor is tacrolimus.

15. Use as described in any one of claims 11 to 14 for production of a preventive and/or therapeutic medicine for rheumatoid arthritis, wherein the preventive and/or therapeutic medicine for rheumatoid arthritis is for oral administration.

## Fig 1

| | Control group | Drug A 3mg/kg | Drug A 3mg/kg + Drug B 0.3mg/kg | Drug B 0.1mg/kg | Drug B 0.3mg/kg | Drug B 1mg/kg |
|---|---|---|---|---|---|---|
| Percent edema suppression (%) | | 18.5% | 30.9% | 1.4% | 11.2% | 26.4% |

## INTERNATIONAL SEARCH REPORT

| | International application No. |
|---|---|
| | PCT/JP2006/321466 |

A. CLASSIFICATION OF SUBJECT MATTER
*A61K45/06*(2006.01)i, *A61K31/439*(2006.01)i, *A61K31/50*(2006.01)i, *A61P19/02*
(2006.01)i, *A61P29/00*(2006.01)i

According to International Patent Classification (IPC) or to both national classification and IPC

B. FIELDS SEARCHED

Minimum documentation searched (classification system followed by classification symbols)
A61K45/06, A61K31/439, A61K31/50, A61P19/02, A61P29/00

Documentation searched other than minimum documentation to the extent that such documents are included in the fields searched
Jitsuyo Shinan Koho        1922-1996   Jitsuyo Shinan Toroku Koho   1996-2007
Kokai Jitsuyo Shinan Koho  1971-2007   Toroku Jitsuyo Shinan Koho   1994-2007

Electronic data base consulted during the international search (name of data base and, where practicable, search terms used)
BIOSIS(STN), CAplus(STN), EMBASE(STN), MEDLINE(STN), REGISTRY(STN),
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| A | WO 99/25697 A1 (Kowa Co., Ltd.),<br>27 May, 1999 (27.05.99),<br>& EP 1043317 A1      & US 6348468 B<br>& JP 11-152274 A      & JP 2000-198776 A | 1-5,11-15 |
| A | WO 2004/083188 A1 (Kowa Co., Ltd.),<br>30 September, 2004 (30.09.04),<br>& EP 1604984 A1      & US 2006/142292 A1 | 1-5,11-15 |
| A | WO 2000/50408 A1 (Kowa Co., Ltd.),<br>31 August, 2000 (31.08.00),<br>& EP 1156039 A1      & US 6680316 B<br>& JP 2000-247959 A | 1-5,11-15 |

| ☒ Further documents are listed in the continuation of Box C. | ☐ See patent family annex. |

\* Special categories of cited documents:
"A" document defining the general state of the art which is not considered  to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>11 January, 2007 (11.01.07) | Date of mailing of the international search report<br>23 January, 2007 (23.01.07) |
|---|---|
| Name and mailing address of the ISA/<br>Japanese Patent Office | Authorized officer |
| Facsimile No. | Telephone No. |

Form PCT/ISA/210 (second sheet) (April 2005)

**INTERNATIONAL SEARCH REPORT**

International application No.

PCT/JP2006/321466

C (Continuation).   DOCUMENTS CONSIDERED TO BE RELEVANT

| Category* | Citation of document, with indication, where appropriate, of the relevant passages | Relevant to claim No. |
|---|---|---|
| A | JP 11-349520 A  (The Kitasato Institute),<br>21 December, 1999 (21.12.99),<br>(Family: none) | 1-5,11-15 |
| A | Kiyoshi UDA "Tacrolimus no Ko Rheumatism Sayo ni Kansuru Kenkyu", Heisei 15 Nendo Kousei Rodo Kagaku Kenkyuhi Hojokin Men'eki Allergy Shikkan Yobo Chiryo Kenkyu Jigyo Kenkyu Hokokusho, Dai 1 Bunsatsu,  2004,<br>pages 20 to 22 | 1-5,11-15 |
| A | Kiyoshi UDA et al., "FK 506 (Tacrolimus) no Yakuri Sayo", , Medicine and drug journal, Vol.36, No.4, 2000, pages 106 to 111 | 1-5,11-15 |
| A | Sakuma S. et al., FK506 potently inhibits T cell activation induced TNF-$\alpha$ and IL-$\beta$ production in vitro by human peripheral blood mononuclear cells, British Journal of Pharmacology, Vol.130, No.7, 2000, p.1655-1663 | 1-5,11-15 |
| A | WO 2004/069267 A1  (Novartis AG.),<br>19 August, 2004 (19.08.04),<br>& EP 1594522 A1          & US 2006/83854 A1<br>& JP 2006-517217 A | 1-5,11-15 |

Form PCT/ISA/210 (continuation of second sheet) (April 2005)

## INTERNATIONAL SEARCH REPORT

| International application No. |
| --- |
| PCT/JP2006/321466 |

**Box No. II**     **Observations where certain claims were found unsearchable (Continuation of item 2 of first sheet)**

This international search report has not been established in respect of certain claims under Article 17(2)(a) for the following reasons:

1. ☒ Claims Nos.: 6-10
because they relate to subject matter not required to be searched by this Authority, namely:
Claims 6 to 10 pertain to methods for treatment of the human body by therapy and thus relate to a subject matter which this International Searching Authority is not required, under the provisions of the PCT Rule 39.1(iv), to search.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable,
the              payment of a protest fee..

                       ☐ The additional search fees were accompanied by the applicant's protest but the applicable protest fee was not paid within the time limit specified in the invitation.

Form PCT/ISA/210 (continuation of first sheet (2)) (April 2005)

| INTERNATIONAL SEARCH REPORT | International application No. |
|---|---|
| | PCT/JP2006/321466 |

<Subject of search>

Claims 1-5 and 11-15 relate to a prophylactic and/or therapeutic agent for rheumatoid arthritis, which comprises a combination of compounds respectively defined by desired properties "an IL-1β inhibitor" and "a calcineurin inhibitor", and claims 1-5 and 11-15 include within their scopes any combination of the compounds having the properties. However, those combinations which are supported by the description in the meaning within PCT Article 6 and disclosed in the meaning within PCT Article 6 are limited to an extremely small part of the claimed combinations.

Further, with respect to the properties "an IL-1β inhibitor" and "a calcineurin inhibitor", even though the common technical knowledge at the time of the filing of the present application is taken into the consideration, it appears that the scopes of the compounds respectively having the properties cannot be specified. Thus, claims 1-5 and 11-15 do not comply with the requirement of clearness under PCT Article 6, too.

Such being the case, the search was made on the relationship between the use of a combination of "an IL-1β inhibitor" and "a calcineurin inhibitor" and a prophylactic and/or therapeutic agent for rheumatoid arthritis and also made on a prophylactic and/or therapeutic agent for rheumatoid arthritis which comprises a combination of the compounds disclosed in the description specifically and defined in claims 3, 4, 13 and 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 133605
- WO 92011860 A
- WO 00007594 A
- WO 99018120 A
- JP 2000309590 A
- WO 01053353 A
- WO 02016436 A
- WO 04072116 A
- WO 99025697 A
- WO 95029672 A
- WO 90225458 A
- WO 01005745 A
- WO 01042189 A
- WO 01005751 A

### Non-patent literature cited in the description

- *Arthritis & Rheumatism,* 2002, vol. 46, 328-346
- *Proc Natl Acad Sci USA.,* 2004, vol. 101, 7554-7559
- *Arthritis & Rheumatism,* 1999, vol. 42, 498-506
- *J. Pharmacobio-Dyn.,* 1992, vol. 11, 649-655
- *J. Rheumatol.,* 1996, vol. 23, 1778-1783